# EUROPEAN PATENT SPECIFICATION

(11) **EP 2 382 187 B1**
(45) Date of publication and mention of the grant of the patent: **19.03.2014**
(21) Application number: 09775799.1
(22) Date of filing: 18.12.2009
(51) Int. Cl.: C07C 255/52, A61Q 13/00, C07C 45/44

(54) **COMPOUND**
VERBINDUNG
COMPOSÉ

(30) Priority: 22.12.2008 GB 0823316
(43) Date of publication of application: 02.11.2011
(73) Proprietor: Givaudan SA, 1214 Vernier (CH)
(72) Inventor: SCHRÖDER, Fridtjof, CH-8442 Hettlingen (CH); MUELLER, Urs, CH-8600 Duebendorf (CH); FRATER,Georg, CH-8400 Winterthur (CH)
(74) Representative: Givaudan Patents
(86) International application number: PCT/CH2009/000406
(87) International publication number: WO 2010/072008

(56) References cited:
- WO-A-01/60777
- WO-A-2006/086908
- BOROSY, A. ET AL.: TETRAHEDRON, vol. 65, 2009, pages 10495-10505,

## Description

This disclosure relates to organic compounds and to fragrance products obtained therefrom.

There is provided a compound of the formula 1 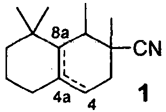 in which the dotted line represents a single double bond in either the 4a,8a- or the 4,4a-position.

The two compounds represented by this formula are 1,2,8,8-tetramethyl-1,2,3,4,5,6,7,8-octahydronaphthalene-2-carbonitrile and (1*RS*,8a*SR*)-1,2,8,8-tetramethyl-1,2,3,5,6,7,8,8a-octahydronaphthalene-2-carbonitrile, shown here as **1β** and **1γ**, respectively (for the α,β,γ-designation of octahydronaphthalenes see G. Ohloff, Riechstoffe & Aromen, 38 - 40, 1957):

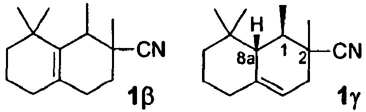

Compound **1**β has two asymmetric centres, and exists as two diastereomers. It may be prepared as a diastereomeric mixture, or it may be prepared selectively as the *cis*-isomer, ***cis***-**1**β:

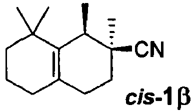

Although compound **1**γ has a third asymmetric centre in its 8a-position, it exists only as two diastereomers, because the methyl group at C(1) and the proton at C(8a) are on the same side of the bicycle (for an explanation of this relation see G. Fráter, F. Schröder, J. Org. Chem. 72, 1112 - 1120, 2007), whereas the nitrile group at C(2) and the methyl group at C(1) can be *cis* or *trans* to each other. Therefore, only the *cis-trans* relation at C(1) and C(2) of the bicycles **1γ** and **5γ** is relevant.

The desired compounds **1**y and *cis*-**1β** may be prepared by a number of different methods, all well known to the art. Some typical (and non-limiting) examples are shown in the following scheme:

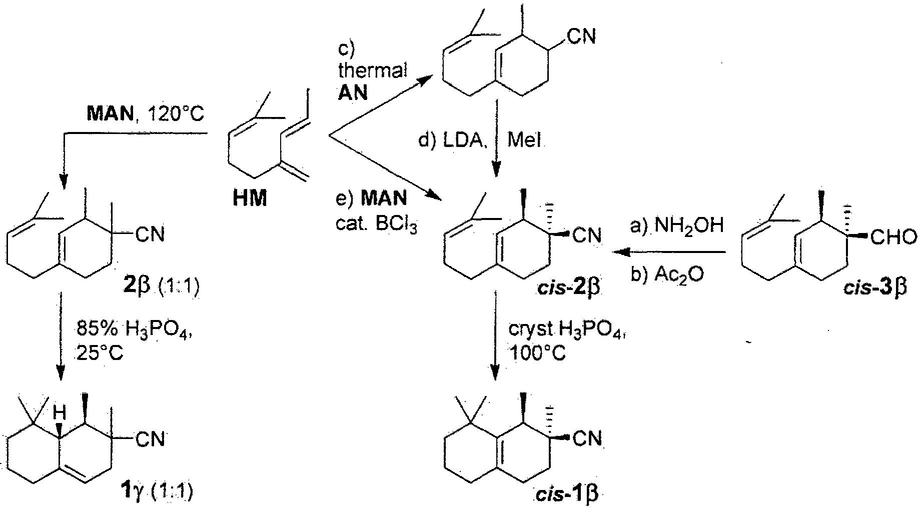

Thus, diastereomeric mixture **1γ** and *cis*-**1β** may be prepared by acid-promoted cyclization of the monocyclic precursors **2β** and ***cis*-2**β respectively. The cyclization of **2**β to **1**γ is carried out under less drastic conditions to prevent double bond isomerization from the y- to the β-position. **2β** may be prepared as *cis*/*trans*-mixture by thermal Diels-Alder reaction of homomyrcene (**HM**) with methacrylonitrile (**MAN**). The preparation of **HM** is known from the literature (J.-P. Barras, B. Bourdin. F. Schröder, Chimia 60, 574-579, 2006). *Cis*-**2β** may be prepared, as shown in the scheme above, by:
a).b) hydroxylamine condensation / elimination from the corresponding aldehyde *cis*-**3β** (G. Fráter, U. Müller, F. Schröder, Tetrahedron: Asymmetry 15, 3967 - 3972 (2003)),
c).d) Diels-Alder reaction of acrylonitrile (**AN**) with **HM**, followed by *cis*-selective α-methylation of the resulting Diels-Alder product, and
e) by Lewis acid-catalyzed Diels-Alder reaction of **HM** with **MAN**.

The nitriles *cis*-**1β** and mixture **1**γ are themselves odorous substances, and may be used as such. However, they are particularly useful as intermediates in the preparation of desirable fragrant substances.

One such desirable material is *cis*-2-acetyl-1,2,3,4,5,6,7,8-octahydro-1,2,8,8-tetramethylnaphthalene, the olfactorily most potent isomer of the commercial compound Georgywood^{™} (see, for example, Fráter, Müller and Schröder, Tetrahedron: Asymmetry, 15 (2004), 3967-3972 and G. Fráter and F. Schröder, J. Org. Chem. 72. 1112 - 1120, 2007) and references therein) as shown in Formula **4** below: 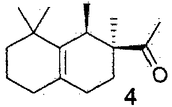 preparable from ***cis***-**1β**.

Another desirable fragrance compound is commercially available as Iso E Super^{™}, which consists mainly of isomer mixture **5a**, but also contains other structural isomers such as **5b**.

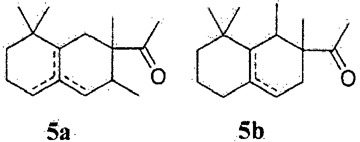

It has been established that the olfactory vector of mixture **5** is the so-called "Super Plus" isomer, 1-((1*RS*,2*RS*,8a*SR*)-1,2,8,8-tetramethyl-1,2,3,5,6,7,8,8a-octahydronaphthalen-2-yl)ethanone, as shown in Formula **5c** below: 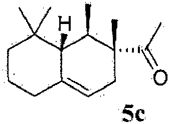 (see C. Nussbaumer, G. Fráter, P. Kraft, Helv. Chim. Acta 82, 1016 - 1024, 1999).

When prepared from the mixture **1**γ, (1RS,8aSR)-1,2,8,8-tetramethyl-1,2,3,5,6,7,8,8a-octahydronaphthalene-2-carbonitrile, the mixture of all isomers 5 has a relatively high content (up to 40%) of the desirable isomer **5c**, which is generated from the compound ***trans*-1**γ:

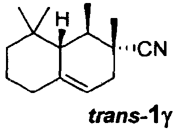

The selective synthesis of **5c** by other methods is difficult (see M. Erman, P. Whelan, C. Cárdenas, M. Antipin and A. N. Nesmeyanov, Perfumer & Flavorist 26, 16 - 21, 2001 and references therein. See also M. Erman, M. J. Williams, C. Cardenas, WO 0160777, Millenium, 2000).

As fragrance materials in their own right, the compounds of Formula **1** may be used alone or in combination with known odorant molecules selected from the extensive range of natural and synthetic molecules currently available, such as essential oils and extracts, alcohols, aldehydes and ketones, ethers and acetals, esters and lactones, macrocycles and heterocycles, and/or in admixture with one or more ingredients or excipients conventionally used in conjunction with odorants in fragrance compositions, for example, carrier materials, and other auxiliary agents commonly used in the art, e.g., solvents such as dipropylene glycol, isopropylmyristate, and triethylcitrate.

Non-limiting examples of known odorant molecules, which may be combined with the compounds of Formula 1 include:
- essential oils and extracts, e.g. oak moss absolute, basil oil, tropical fruit oils, such as bergamot oil and mandarine oil, mastic absolute, myrtle oil, palmarosa oil, patchouli oil, petitgrain oil, wormwood oil, lavender oil, rose oil, jasmin oil, ylang-ylang oil and sandalwood oil.
- alcohols, e.g. cis-3-hexenol, cinnamic alcohol, citronellol, Ebanol^{®}, eugenol, farnesol, geraniol, menthol, nerol, rhodinol, Super Muguet^{™}, linalool, phenylethyl alcohol, Sandalore^{®}, terpineol and Timberol^{®} (1-(2,2,6-Trimethylcyclohexyl)hexan-3-ol).
- aldehydes and ketones, e.g. citral, hydroxycitronellal, Lilial^{®}, methylnonylacetaldehyde, anisaldehyde, allylionone, verbenone, nootkatone, geranylacetone, □-amylcinnamic aldehyde, Georgywood^{™}, hydroxycitronellal, Iso E Super^{®}, Isoraldeine^{®} (methylionone), Hedione^{®}, maltol, methyl cedryl ketone, and vanillin.
- ethers and acetals, *e.g.* Ambrox^{®}, geranyl methyl ether, rose oxide or Spirambrene^{®}.
- esters and lactones, e.g. benzyl acetate, cedryl actetate, □-decalactone, Helvetolide^{®}, □-undecalactone, vetivenyl acetate, cinnamyl propionate, citronellyl acetate, decyl acetate, dimethylbenzylcarbinyl acetate, ethyl acetoacetate, cis-3-hexenyl isobutyrate, linalyl acetate and geranyl acetate.
- macrocycles, *e.g.* ambrettolide, ethylene brassylate or Exaltolide^{®}.
- heterocycles, *e.g.* isobutylquinoline.

The compounds of Formula 1 may be used in a broad range of fragrance applications, e.g. in any field of fine and functional perfumery, such as perfumes, household products, laundry products, body care products and cosmetics. The compounds can be employed in widely varying amounts, depending upon the specific application and on the nature and quantity of other odorant ingredients. The proportion is typically from 0.0001 to 2 weight percent of the application. In one embodiment, compounds of the present invention may be employed in a fabric softener in an amount of from 0.0001 to 0.005 weight percent. In another embodiment, compounds of the present invention may be used in an alcoholic solution in amounts of from 0.01 to 3 weight percent, more preferably between 0.5 and 2 weight percent. However, these values are given only by way of example, since an experienced perfumer may also achieve effects or may create novel accords with lower or higher concentrations, e.g. up to about 20 weight percent based on the fragrance composition.

The compounds of Formula **1** may be employed into the fragrance application simply by directly mixing the fragrance composition with the fragrance application, and/or they may, in an earlier step, be entrapped with an entrapment material such as polymers, capsules, microcapsules and nanocapsules, liposomes, film formers, absorbents such as carbon or zeolites, cyclic oligosaccharides and mixtures thereof, and/or they may be chemically bonded to substrates, which are adapted to release the fragrance molecule upon application of an external stimulus such as light, enzyme, or the like, and then mixed with the application.

Thus, there is additionally provided a method of manufacturing a fragrance application and consumer products resulting therefrom, comprising the incorporation therein of at least one compound of formula **1** as a fragrance ingredient, either by directly admixing the compound to the application, or by admixing a fragrance composition comprising at least one compound of formula **1** or a precursor thereof, which may then be mixed to a fragrance application, using conventional techniques and methods. Through the addition of an olfactorily-acceptable amount of a compound of the present invention, the odour notes of a fragrance application will be improved, enhanced or modified.

There is therefore additionally provided a method for improving, enhancing or modifying a fragrance application by means of the addition thereto of an olfactorily-acceptable amount of at least one compound of formula **1**.

There is additionally provided a fragrance application comprising:
a) an odorant comprising at least one compound of formula 1; and
b) a consumer product base.

As used herein, "fragrance application" means any consumer product, including, but not limited to, fine fragrances, *e.g.* eaux de perfume and eaux de toilette; household products, *e.g.* detergents for dishwasher, surface cleaner, air freshener; laundry products, *e.g.* softener, bleach, detergent; body care products, *e.g.* after-shave lotion, shampoo, shower gel, shower and bath salt, hygiene product; and cosmetics, *e.g.* deodorants, vanishing creams, comprising an odorant. This list of products is given by way of illustration and is not to be regarded as being in any way limiting.

As used herein, "consumer product base" means a composition for use as a consumer product to fulfil specific actions, such as cleaning, softening, and caring or the like. Examples of such products include fabric care and personal care products such as laundry care detergents, rinse conditioners, personal cleansing compositions. The composition may comprise a variety of active ingredients such as surfactants, polymers, fillers and auxiliary agents, such as dyes and solvents.

However, as previously mentioned, the compounds are especially useful for the preparation of high-performance fragrance molecules. There is therefore provided a method for the preparation of *cis*-2-acetyl-1,2,3,4,5,6,7,8-octahydro-1,2,8,8-tetramethylnaphthalene, comprising the methylation of ***cis***-1β with an appropriate organometallic compound such as methylmagnesium halides or methyllithium followed by hydrolysis of the thus-formed imine intermediate.

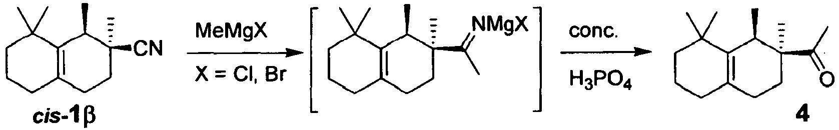

There is further provided a method for the preparation of 5b having a relatively high content (up to 40%) of an isomer of the formula 5c, 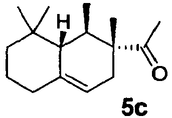 comprising the methylation of nitrile **1**γ with an appropriate organometallic compound such as methylmagnesium halides or methyllithium und subsequent hydrolysis of the thus-formed imine.

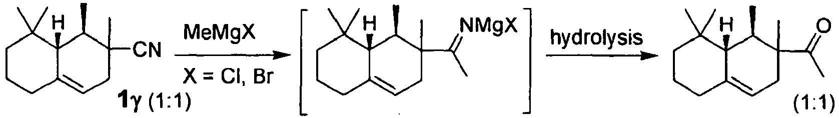

The disclosure is further described with reference to the following non-limiting examples.

### EXAMPLE 1

### Cis-1,2-dimethyl-4-(4-methylpent-3-enyl)cyclohex-3-enecarbonitrile 2β (via Oxime):

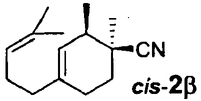

*Cis*-1,2-dimethyl-4-(4-methylpent-3-enyl)cyclohex-3-enecarbaldehyde **3**β (G. Fráter, U. Müller, F. Schröder, Tetrahedron: Asymmetry 15, 3967 - 3972, 2004) (50 g, 0.23 mol) in ethanol (250 ml) is added slowly to NH₂OH x HCl (24 g, 0.35 mol) and Na₂CO₃ (24 g, 0.23 mol) in water (60 ml). The mixture is heated to 60 - 70°C for 1 h, then poured upon water (0.5 1). Extraction with tert-butyl methyl ether (0.5 1), washing of the combined organic phases with water and conc. NaCl, drying over MgSO₄, filtration and evaporation of the solvent gives the crude oxime (62 g). For analytical measurements 2 g of the crude are purified by bulb-to-bulb distillation giving 1.75 g (99%) of pure oxime. Analytical data: ¹H-NMR (CDCl₃, 400 MHz): δ 0.9 (d, 3 H), 1.1 (s, 3 H), 1.6 (s, 3 H), 1.6 (m, 1 H), 1.7 (s, 3 H), 1.75 (m, 1 H), 2.9 (4 H), 2.1 (3 H), 5.1 (m, 1 H), 5.2 (s, 1 H), 8.8 (br, 1 H) ppm. ¹³C-NMR (CDCl₃, 400 MHz): 17.1 (q), 17.7 (q), 23.3 (q), 25.7 (q), 25.7 (t), 26.4 (t), 32.8 (t), 37.2 (t), 38.2 (s), 39.3 (d), 124.15 (d), 125.6 (d), 131.5 (s), 136.3 (s), 137.1 (s), 156.5 (d) ppm. GC/MS: 218 (2%, [M -OH]⁺), 217 (1%, [M - H₂O]⁺), 202 (3%, [M - H₂O - CH₃]⁺, 174 (8%), 161 (4%), 149 (5%), 134 (6%), 121 (5%), 107 (20%), 69 (100%), 53 (12%), 41 (70%). IR (film): 3318 (br), 2965 (m), 2916 (m), 1449 (m), 1374 (m), 1305 (w), 1102 (w), 943 (s), 836 (w), 748 (w).

The remaining crude oxime (60 g, 0.22 mol) in toluene (20 ml) is added dropwise over 20 min to acetanhydride (102 g, 1 mol) and toluene (130 ml) at reflux. Then water (20 ml) is added at 70-80°C. After extraction with hexane (100 ml), washing of the organic phase with conc. KHCO₃ and water the organic phase is dried over MgSO₄, filtered and the solvents are evaporated. The residue is purified by flash chromatography over 1.2 kg silicagel using hexane / *tert*-butyl methyl ether 4:1 as eluent. After evaporation of the solvents the residue is purified by distillation giving 31 g (65%) of ***cis*-2**β as an oil. Analytical data of ***cis*-2**β: ¹H-NMR (CDCl₃, 400 MHz): 1.2 (d, 3 H), 1.4 (s, 3 H), 1.65 (s, 3 H), 1.7 (s, 3 H), 2.0 (5H), 2.1 (3 H), 2.3 (1 H), 5.1 (2 H) ppm. ¹³C-NMR (CDCl₃, 400 MHz): δ 17.4 (q), 17.7 (q), 24.7 (q), 25.65 (q), 26.1 (t), 26.3 (t), 33.7 (t), 37.05 (t), 37.1 (s), 38.95 (d), 122.8 (s), 123.9 (d), 124.0 (d), 131.6 (s), 137.1 (s) ppm. GC/MS: 217 (5%, M⁺), 202 (6%, [M - 15]⁺), 174 (13%), 149 (7%), 134 (7%), 107 (13%), 69 (100%), 41 (28%). IR (film): 2969 (s), 2926 (s), 2857 (w), 2233 (w, CN), 1669 (w), 1453 (s), 1376 (m), 1343 (w), 1287 (w), 1173 (w), 1145 (w), 1105 (w), 984 (w), 885 (w), 829 (m), 635 (w), 611 (w), 603 (w). Odour: anisic, earthy, calamus, agrestic.

### EXAMPLE 2

### Cis-1,2-dimethyl-4-(4-methylpent-3-enyl)cyclohex-3-enecarbonitrile 2β (via BCl₃-catalyzed MANDA reaction):

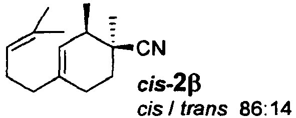

Methacrylonitrile (111 g, 1.65 mol) is added dropwise at 10 - 20°C to boron trichloride 1M in xylene (225 ml, 0.22 mol). Freshly distilled homomyrcene of 74% purity (225 g, 1.1 mol) is added at 10 - 20°C within 40 min. The reaction is kept for another 40 min at 10 - 20°C, then for 3h at 25°C. After complete conversion the reaction mass is poured upon ice-cold NaHCO₃ and extracted with tert-butyl methyl ether. The combined organic phase is washed with NaHCO₃ and dried over MgSO₄. Filtration and evaporation under reduced pressure gives 306 g of a red oil. After addition of 65 g paraffin oil, 15 g Na₂CO₃ and 0.2 g hydroquinone the residue is short-path-distilled giving pre-fractions at 69°C / 0.05 Torr and 214 g of **2**β at 105°C / 0.05 Torr. Yield: 77 % (dist, corr, based on purity of the homomyrcene and the *cis*-isomer). GC/MS: 8.4 (86%, *cis*), 8.6 (14%, *trans*) min. The analytical data of the main isomer (*cis*) are identical with the ones obtained for ***cis*-2**β (Example 1).

### EXAMPLE 3

### 1,2-dimethyl-4-(4-methylpent-3-enyl)cyclohex-3-enecarbonitrile 2β (by thermal DA):

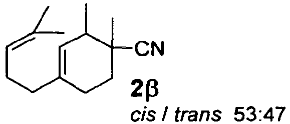

Methacrylonitrile (44 g, 0.66 mol), freshly distilled homomyrcene of 74% purity (100 g, 0.51 mol) and hydroquinone monomethyl ether (0.1 g) are heated at reflux (130°C) for 36 h. After cooling to r.t. the reaction mass is distilled at 0.05 mbar giving 22 g pre-fractions at 25 - 75°C and 71 g (65% d. Th.) of **2**β at 130°C. GC/MS; 8.4 (53%, *cis*), 8.6 (47%, *trans*) min.

The analytical data of the *cis*-isomer are identical with the ones obtained for ***cis*-2**β (Example 1). For the analysis of the relative configuration of ***trans*-2**β this isomer was separated from the mixture by preparative GC and its structure proven by NMR-analysis (HSQC, HMBC, COSY and NOESY) in C₆D₆. Analytical data of ***trans***-**2**β: ¹H-NMR (C₆D₆, 500 MHz): δ 0.72 (d, *J*=7.05 Hz, 3 H), 0.82 (s, 3 H), 1.36 (m, 1 H), 1.48 (m, 3 H), 1.5 (s, 3 H), 1.55 (m, 1 H), 1.65 (d, *J*=1.07 Hz, 3 H), 1.78 (m, 1 H), 1.84 (t, *J*=7.48 Hz, 2 H), 2.03 (m, 2 H), 2.28 (dd, *J*=7.0 5, 1.71 Hz, 1 H), 4.94 (m, 1 H), 5.11 (m, 1 H) ppm. ¹³C-NMR (C₆D₆, 500 MHz): δ 16.2 (q), 17.7 (q), 18.5 (q), 25.0 (t), 25.8 (q), 26.6 (t), 31.5 (t), 34.4 (s), 37.4 (d), 37.4 (t), 123.9 (d), 124.4 (d), 125.2 (s), 131.5 (s), 136.2 (s) ppm. GC/MS: 217 (4%, M⁺), 202 (5%, [M - 15]⁺), 174 (15%), 149 (5%), 134 (7%), 121 (5%), 107 (14%), 69 (100%), 41 (29%).

### EXAMPLE 4

### Cis-1,2,8,8-tetramethyl-1,2,3,4,5,6,7,8-octahydronaphthalene-2-carbonitrile 1β:

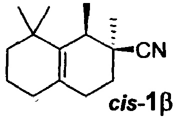

*Cis*-1,2-dimethyl-4-(4-methylpent-3-enyl)cyclohex-3-enecarbonitrile **2**β (55 g, 0.25 mol) is added within 10 min to crystalline H₃PO₄ (22 g, 0.22 mol) molten at 105°-120°C under stirring and nitrogen. After another 15 min at this temperature the mixture is cooled and water (150 ml) is added at 80°C. After phase separation and extraction with hexane the combined organic phases are washed with conc. NaHCO₃ to pH 8. Drying over MgSO₄, filtration and concentration under reduced pressure gives 55 g of an orange oil, which is short-path-distilled giving 51.5 g of ***cis*-1**β at 84°C / 0.04 Torr. Yield: 90 % (dist, corr, based on the *cis*-isomer). GC/MS: 8.7 (12%, ***trans*-1**β), 8.9 (6%, ***cis*-1**γ), 9.0 (81%, ***cis*-1**β) min. Analytical data of ***cis*-1**β: ¹H-NMR (CDCl₃, 400 MHz): 0.96 (s, 3 H) 1.07 (s, 3 H) 1.28 (s, 3 H), 1.3 (s, 3 H) 1.47 (m, 2 H) 1.66 (m. 2 H), 1.65 - 1.95 (4 H), 2.01 (m, 1 H) 2.15 (ddd, 1H) 2.27 (m, 1 H) ppm. ¹³C-NMR (CDCl₃, 400 MHz): δ 19.05 (t), 20.62 (q), 22.65 (q), 26.25 (t), 26.30 (t), 28.31 (q), 29.47 (q), 30.82 (t), 34.04 (s), 36.13 (d), 36.44 (s), 40.05 (t), 125.44 (s), 125.67 (s), 135.93 (s) ppm. GC/MS: 217 (11%, M⁺), 202 (100%, [M - 15]⁺), 175 (21%), 135 (28%), 121 (10%), 107 (12%), 105 (10%), 91 (14%), 79 (7%), 77 (11%), 75 (10%), 41 (11%). IR (film): 2930 (s), 2836 (m), 2231 (w), 1459 (s), 1379 (s), 1361 (w), 1281 (w), 1261 (w), 1235 (w), 1204 (w), 1180 (w), 1166 (w), 1114 (w), 1068 (w), 1029 (w), 995 (w), 945 (w), 874 (w), 843 (w), 645 (w). Odour: woody, cedarwood with a sweet floral touch, earthy.

### EXAMPLE 5

### (1RS,8aSR)-1,2,8,8-tetramethyl-1,2,3,5,6,7,8,8a-octahydronaphthalene-2-carbonitrile 1γ:

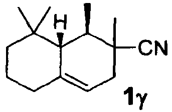

1,2-Dimethyl-4-(4-methylpent-3-enyl)cyclohex-3-enecarbonitrile **2**β (50 g, 0.216 mol) and 85% H₃PO₄ (25 g, 0.216 mmol) are stirred for 5 days at 25°C. The reaction mass is poured upon conc. NaHCO₃ After phase separation and extraction with *tert*-butyl methyl ether the combined organic phase is washed with water, dried over MgSO₄, filtered and concentrated under reduced pressure giving 46 g of a yellow oil, which is short-path-distilled giving 46 g (80% corr) of **1**γ. GC/MS: 8.7 (12%, ***cis*-1**β), 8.9 (46%, ***cis*-1**γ), 9.0 (36%, ***trans*-1**γ) min. The analytical data of ***cis*-1**γ are identical with the ones of this compound prepared above (Example 4). Analytical data of ***trans*-1**γ: ¹H-NMR (CDCl₃, 400 MHz): δ 0.81 (s, 3 H), 1.04 (s, 3 H), 1.19 (s, 3 H), 1.25 (d, 3 H), 1.4 - 2.4 (10 H), 5.35 (d, 1 H) ppm. ¹³H-NMR (CDCl₃, 400 MHz): δ 17.8 (q), 20.13 (q), 20.17 (q), 23.75 (t), 31.62 (q), 35.98 (d), 36.16 (t), 37.45 (d), 42.9 (t), 52.5 (d), 115.0 (d), 126.5 (s), 141.07 (s) ppm. GC/MS: 217 (12%, M⁺), 202 (42%, [M -15]⁺), 174 (32%), 161 (14%), 149 (14%), 134 (11%), 107 (19%), 91 (20%), 69 (100%), 55 (12%), 41 (29%). GC/MS: 217 (10%, M⁺), 202 (13%, [M - 15]⁺), 174 (30%), 161 (10%), 107 (13%), 91 (16%), 69 (100%), 41 (29%).

## Claims

1. A compound of the formula 1 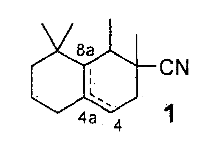 in which the dotted line represents a single double bond in either the 4a,8a- or the 4,4a-position.

2. A compound of claim 1, in which the compound is 1,2,8,8-tetramethyl-1,2,3,4,5,6,7,8-octahydronaphthalene-2-carbonitrile.

3. A compound of claim 1, in which the compound is (*1RS,8aSR*)-1,2,8,8-tetramethyl-1,2,3,5,6,7,8,8a-octahydronaphthalene-2-carbonitrile.

4. A method of improving, enhancing or modifying a fragrance application by means of the addition thereto of an olfactorily-acceptable amount of at least one compound according to claim 1.

5. A fragrance application comprising:
a. an odorant comprising at least one compound according to claim1; and
b. a consumer product base.

6. A method of preparing *cis*-2-acetyl-1,2,3,4,5,6,7,8-octahydro-1,2,8,8 tetramethylnaphthalene, comprising the methylation of *cis*-1,2,8,8-tetramethyl-1,2,3,4,5,6,7,8-octahydronaphthalene-2-carbonitrile with an appropriate organometallic compound, followed by hydrolysis of the imine intermediate thus formed.

7. A method of preparing 1-((1RS,2RS,8aSR)-1,2,8,8-tetramethyl-1,2,3,5,6,7,8,8a-octahydronaphthalen-2-yl)ethanone, comprising the methylation of (1*RS*,8a*SR*)-1,2,8,8-tetramethyl-1,2,3,5,6,7,8,8a-octahydronaphthalene-2-carbonitrile with an organometallic compound and subsequent hydrolysis of the imine thus formed.

## Patentansprüche

1. Verbindung der Formel I 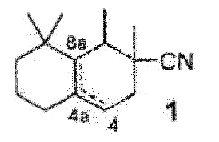 wobei die gestrichelte Linie für eine einzige Doppelbindung entweder in der 4a,8a-Stellung oder in der 4,4a-Stellung steht.

2. Verbindung nach Anspruch 1, wobei es sich bei der Verbindung um 1,2,8,8-Tetramethyl-1,2,3,4,5,6,7,8-octahydronaphthalin-2-carbonitril handelt.

3. Verbindung nach Anspruch 1, wobei es sich bei der Verbindung um (*1RS,8aSR*)-1,2,8,8-Tetramethyl-1,2,3,5,6,7,8,8a-octahydronaphthalin-2-carbonitril handelt.

4. Verfahren zur Verbesserung, Verstärkung oder Modifizierung einer Duftstoffanwendung durch Zugabe einer olfaktorisch annehmbaren Menge mindestens einer Verbindung nach Anspruch 1.

5. Duftstoffanwendung, umfassend:
a. ein Odorans, umfassend mindestens eine Verbindung nach Anspruch 1; und
b. eine Konsumproduktbasis.

6. Verfahren zur Herstellung von *cis*-2-Acetyl-1,2,3,4,5,6,7,8-octahydro-1,2,8,8-tetramethylnaphthalin, bei dem man *cis*-1,2,8,8-Tetramethyl-1,2,3,4,5,6,7,8-octahydronaphthalin-2-carbonitril mit einer geeigneten metallorganischen Verbindung methyliert und dann das so gebildete Imin-Zwischenprodukt hydrolysiert.

7. Verfahren zur Herstellung von 1-((1RS,2RS,8aSR)-1,2,8,8-Tetramethyl-1,2,3,5,6,7,8,8a-octahydronaphthalin-2-yl)ethanon, bei dem man (*1RS*,*8aSR*)-1,2,8,8-Tetramethyl-1,2,3,5,6,7,8,8a-octahydronaphthalin-2-carbonitril mit einer metallorganischen Verbindung methyliert und dann das so gebildete Imin hydrolysiert.

## Revendications

1. Composé de formule 1 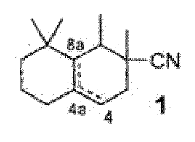 dans laquelle la ligne pointillée représente une seule double liaison soit dans la position 4a,8a, soit dans la position 4,4a.

2. Composé selon la revendication 1, dans lequel le composé est le 1,2,8,8-tétraméthyl-1,2,3,4,5,6,7,8-octahydronaphtalène-2-carbonitrile.

3. Composé selon la revendication 1, dans lequel le composé est le (*1RS,8aSR*)-1,2,8,8-traméthyl-1,2,3,5,6,7,8,8a-octahydronaphtalène-2-carbonitrile.

4. Procédé pour perfectionner, améliorer ou modifier une application de parfum au moyen de l'addition à celui-ci d'une quantité acceptable au plan olfactif d'au moins un composé selon la revendication 1.

5. Application de parfum comprenant :
a. une substance odorante comprenant au moins un composé selon la revendication 1 ; et
b. une base de produit de consommation.

6. Procédé de préparation du *cis*-2-acétyl-1,2,3,4,5,6,7,8-octahydro-1,2,8,8-tétraméthylnaphtalène, comprenant la méthylation du *cis*-1,2,8,8-tétraméthyl-1,2,3,4,5,6,7,8-octahydronaphtalène-2-carbonitrile à l'aide d'un composé organométallique approprié, suivie de l'hydrolyse de l'intermédiaire imine ainsi formé.

7. Procédé de préparation de la 1-((*1RS,2RS,8aSR*)-1,2,8,8-tétraméthyl-1,2,3,5,6,7,8,8a-octahydronaphtalén-2-yl)éthanane, comprenant la méthylation du (*1RS,8aSR*)-1,2,8,8-tétraméthyl-1,2,3,5,6,7,8,8a-octahydronaphtalène-2-carbonitrile à l'aide d'un composé organométallique et l'hydrolyse consécutive de l'imine ainsi formée.
